# EUROPEAN PATENT SPECIFICATION

(11) **EP 4 293 096 B1**
(45) Date of publication and mention of the grant of the patent: **25.11.2026**
(21) Application number: 23207425.2
(22) Date of filing: 12.10.2018
(51) Int. Cl.: C11C 3/02, C12P 7/64, C12N 9/20, A23L 33/115, C11C 1/04

(54) **PROCESSED OIL COMPRISING MONOACYLGLYCERIDES**
VERFAHREN ZUR HERSTELLUNG VON MONOACYLGLYCERIDÖLEN UND NAHRUNGSMITTELPRODUKTEN MIT MONOACYLGLYCERIDÖLEN
PROCÉDÉ DE FABRICATION D'HUILES MONOACYLGLYCÉRIDES ET PRODUITS ALIMENTAIRES CONTENANT DES HUILES MONOACYLGLYCÉRIDES

(30) Priority: 13.10.2017 US 201762571910 P
(43) Date of publication of application: 20.12.2023
(62) Document number(s) of the earlier application(s) in accordance with Art. 76 EPC: 18865493.3 / 3 694 962
(73) Proprietor: GlycosBio Inc., Houston, TX 77021 (US)
(72) Inventor: MONTICELLO, Daniel J., Texas, 77381 (US); BUSSMANN, Werner J., Texas, 77062 (US)
(74) Representative: Gill Jennings & Every LLP

(56) References cited:
- EP-A1- 2 287 325
- WO-A1-2013/114178
- ROXANA ROSU ET AL: "Repeated Use of Immobilized Lipase for Monoacylglycerol Production by Solid-Phase Glycerolysis of Olive Oil", JAOCS, vol. 74, no. 4, 1 January 1997 (1997-01-01), pages 445 - 450, XP055592138, DOI: 10.1007/s11746-997-0104-2
- ZHU Q ET AL: "A two-stage enzymatic process for synthesis of extremely pure high oleic glycerol monooleate", ENZYME AND MICROBIAL TECHNOLOGY, STONEHAM, MA, US, vol. 48, no. 2, 8 February 2011 (2011-02-08), pages 143 - 147, XP027585727, ISSN: 0141-0229, [retrieved on 20101030]
- GERALD P MCNEILL SHOICHI SHIMIZU TSUNEO YAMANE: "High-yield enzymatic glycerolysis of fats and oils", JOURNAL OF THE AMERICAN OIL CHEMISTS SOCIETY, SPRINGER, DE, vol. 68, no. 1, 1 January 1991 (1991-01-01), pages 1 - 5, XP008185804, ISSN: 0003-021X, DOI: 10.1007/BF02660298
- JIANG Y ET AL: "Magnetic nanoparticles supported ionic liquids for lipase immobilization: Enzyme activity in catalyzing esterification", JOURNAL OF MOLECULAR CATALYSIS B : ENZYMATIC,, vol. 58, no. 1-4, 1 June 2009 (2009-06-01), pages 103 - 109, XP026062997, ISSN: 1381-1177, [retrieved on 20081211], DOI: 10.1016/J.MOLCATB.2008.12.001

## Description

### CROSS-REFERENCE TO RELATED APPLICATIONS

### BACKGROUND

Chronic deficiency in the secretion of digestive enzymes by the pancreas is termed Exocrine Pancreatic Insufficiency (EPI). Without these digestive enzymes, patients suffering from EPI cannot properly digest nutrients in food and may suffer from malnutrition and abdominal disorders. EPI is prevalent in individuals with chronic pancreatitis and several other chronic gastrointestinal disorders. EPI also manifests in patients suffering from cystic fibrosis. The effects of EPI can be mitigated by Pancreatic Enzyme Replacement Therapy (PERT), in which the individual administers enzyme capsules each time food is consumed. Conventionally, PERT treatment comprises pancreatic enzymes extracted from porcine pancreas.

Lipids are energy-dense compounds that are the source of essential long chain fatty acids. Consumed lipids are digested with lipases secreted from the pancreas into free fatty acids (FFA) and monoacylglyceride (MAG). Blockage of lipase release from the pancreas results in very poor digestion of fats and oils. For patients suffering from EPI, this can lead to significant malnutrition because the calories, essential fatty acids and fat-soluble nutrients are trapped in the un-digested lipid particles and pass through the system.

There is an un-met clinical need for alternative sources of nutrition that can be consumed without needing to supplement with PERT by individuals with EPI.

Partially hydrolyzed fats and oils, in the form of MAGs are readily absorbed by individuals with EPI, without the requirement of PERT. MAG oil-based products have been evaluated in the clinic as capsule-based nutritional supplements; however, capsules were utilized to avoid the bad taste. For conventional sources of MAG oils, the starting oil is treated chemically or enzymatically to make MAGs, which are then extracted with solvents and distilled to fractionate the MAGs away from other components of the starting oils. These MAG products are sold as relatively pure products containing only small amounts of contaminating FFA, DAGs and TAGs, and with virtually no other compounds. Thus, conventional sources of MAG oil often lack the other natural compounds found in the oil, such as tocopherol XP055592138 describes the use of immobilized lipase for production of monoacylglycerol (MAG) by solid-phase glycerolysis of fats and oils. Example 1 of WO 2013/114178 A1 describes the hydrolysis of oil with a 80-88% conversion in 24h in two reaction steps: a. Formation of fatty acids and monoglycerides, and b. Formation of fatty acids and glycerol. XP027585727 shows a study about the synthesis of high oleic glycerol monooleate by enzymatic glycerolysis of high oleic sunflower oil, using Novozyme 435 as the biocatalyst, in a binary solvent mixture of tert-butanol and tert-pentanol (80/20, v/v), at a lab scale.

There is clinical need for nutritional products with very-high-energy caloric density that can be consumed by individuals with inefficient or compromised digestive systems. In addition to individuals with pancreas pathologies (*e.g*., cystic fibrosis, pancreatitis and pancreatic cancer patients) other patients with diagnosed or undiagnosed Exocrine Pancreatic insufficiency (EPI) would benefit from the products. In addition, individuals with bile dysfunction (cholestasis) may benefit from "pre-digested" fats that do not require bile acids for emulsification. There are high calorie "energy bars" and drinks on the market. But, these products are not suitable for individuals who are unable to digest (hydrolyze) the fats in the product. Until now, no one has formulated lipids into liquid (shakes) and solid (bar) forms that are suitable for "PERT-free" use.

Accordingly, there is need for high caloric density foods that can be consumed by individuals with inefficient or compromised digestive systems. The present application describes a method to produce an edible enzyme-modified oil (EMO) that is substantially free of triacylglycerides (TAGs).

### SUMMARY

The present invention is directed to a method for making a monoacylglycerol-enriched oil, according to the appended claims.

In some embodiments, said starting oil is an oil derived from plant, animal or fish origin.

In some embodiments, said first enzyme is lipase AY.

In some embodiments, said first period of time is a period of time sufficient to hydrolyze at least 94% of the TAGs in said starting oil.

In some embodiments, said first period of time is between about 14 hours and 24 hours.

In some embodiments, said step of allowing said reaction mixture to react under conditions sufficient for said first enzyme to hydrolyze said TAGs is performed at a temperature between about 30°C and about 35°C.

In some embodiments, said second enzyme is lipase G.

In some embodiments, said second period of time is a period of time sufficient to result in enrichment of MAGs in the lipid oil phase of about 60% to 95%.

In some embodiments, said second period of time is between about 24 hours and about 72 hours.

In some embodiments, said step of allowing said second reaction mixture to react for a second period of time to yield a lipid oil phase and a glycerol phase is performed at a temperature between about 17°C and 23°C.

In some embodiments, the method further comprises drying said reaction product by applying a vacuum for a third period of time sufficient to remove at least a portion of water from the reaction product.

In some embodiments, said step of drying said reaction product is performed at a temperature between 20°C-30°C.

In some embodiments, said drying step is applied throughout the second period of time.

In some embodiments, said step of inactivating said second enzyme is performed by heating said reaction product.

In some embodiments, said heating is performed at a temperature of at least 70°C for at least 1 hour.

In some embodiments, the final concentration of sodium chloride comprises up to 0.3 weight percent sodium chloride.

In some embodiments, the method further comprises before mixing said lipid reaction product and food-grade glycerol and a second enzyme capable of esterifying FFAs and glycerol, re-establishing a nitrogen atmosphere over said lipid reaction product.

In some embodiments, said steps of removing at least a portion of said aqueous phase and replacing said at least a portion of said aqueous phase with about an equivalent volume of water and waiting a second period of time are repeated before performing said step of collecting said lipid reaction product.

In some embodiments, the method further comprises adding tocopherol to said lipid oil phase after collecting said lipid oil phase.

### BRIEF DESCRIPTION OF THE DRAWINGS AND FIGURES

FIGURE 1 depicts the TLC separation of components of starting vegetable oil, intermediate FFAs, and final MAG oil.
FIGURE 2 depicts the TLC separation of components of starting vegetable oil, intermediate FFAs, and final MAG oil.
FIGURE 3 depicts the distribution of FFA, MAG, DAG, and TAG in "Ensure Original Nutritional Shake" and Enzyme Modified Oil product of the present disclosure ("GBFS").
FIGURE 4 depicts a block flow diagram of the process of manufacturing enzyme modified oil.
FIGURE 5 depicts the distribution of amino acids and peptides in ready-to-drink nutritional drinks and GBFS hydrolyzed pea protein.
FIGURE 6A depicts the NMR spectrum of authentic TAG (tristerin).
FIGURE 6B depicts the NMR spectrum for enzyme modified oil produced from almond oil.
FIGURE 7 depicts the increase in serum triglycerides following ingestion of the EMO-based ready-to-drink shake.
FIGURE 8 depicts the increase in serum triglycerides following ingestion of MAG-based RTDS without PERT.

### DETAILED DESCRIPTION OF THE DISCLOSED SUBJECT MATTER

It is to be understood that both the foregoing general description and the following detailed description are exemplary and explanatory only, and are not restrictive of the invention, as claimed. In this application, the use of the singular includes the plural, the word "a" or "an" means "at least one", and the use of "or" means "and/or", unless specifically stated otherwise. Furthermore, the use of the term "including", as well as other forms, such as "includes" and "included", is not limiting. Also, terms such as "element" or "component" encompass both elements or components comprising one unit and elements or components that comprise more than one unit unless specifically stated otherwise.

"Enriched" in the context of this invention means with an amount higher than in the starting material. For example, a MAG-enriched oil is an oil having a MAG content that is greater than the starting MAG content prior to the enrichment process or that the starting oil has a greater percentage of MAG than the oil possessed prior to the enrichment process. The enrichment process can be by conversion of TAGs to MAGs thereby increasing the MAG content and percentage and decreasing the TAG content or percentage.

A triacylglycerol ("TAG"), also known as a triglyceride, is a glyceride consisting of three fatty acid chains covalently bonded to a glycerol molecule through ester linkages. TAGs may also be classified as having a long or medium chain length. Long chain TAGs contain fatty acids with 14 or more carbons, while medium chain TAGs contain fatty acids with 6 to 12 carbons. Long chain TAGs can include omega-3 and omega-6 fatty acids. Medium chain TAGs have saturated fatty acids and thus do not contain omega-6 or omega-3 fatty acids. Long chain TAGs (LCT) and medium chain triglycerides (MCT) can serve as energy sources.

A diacylglycerol ("DAG"), also known as a diglyceride, is a glyceride consisting of two fatty acid chains covalently bonded to a glycerol molecule through ester linkages.

A monoacylglycerol ("MAG"), also known as a monoglyceride, is a glyceride consisting of one fatty acid chain covalently bonded to a glycerol molecule through an ester linkage
As used herein, the term "processed oil" refers to a non-naturally occurring oil composition substantially free of triacylglycerols ("TAGs") or having a reduced amount of TAGs with respect to the pre-modified or pre-processed oil.

As used herein, the terms "enzyme-modified oil" or "EMO" refers to a processed oil wherein TAGs were enzymatically converted to MAGs, such as, for example, using the enzymatic conversion of the present disclosure.

As used herein, the term "food product" refers to a manufactured or non-naturally occurring food product. It should be understood that the food product referred to herein, while manufactured and non-naturally occurring as a whole, can comprise various combinations of natural ingredients where said combinations either do not occur in nature or where said combinations do exist in nature, they do not exist in the relative amounts used in the food product.

As used herein, a "non-oil ingredient" is an ingredient that is naturally present in an oil source that is not a MAG, DAG, TAG, FFA or lipid.

In some embodiments, the starting oils may comprise, by way of example but not limitation, oils derived from plants such as olive oil, almond oil flaxseed oil, sunflower seed oil, corn oil, rapeseed oil, palm oil, soy bean oil, or oil derived from animals such as fish oil, sardine oil, or anchovy oil, or algal oil, or mixtures thereof. In one aspect, the starting oil comprises a blend of olive oil, sunflower seed oil, and flaxseed oil, wherein the from about 50% to about 80% by weight of the total weight of the starting oil is olive oil, from about 10% to about 30% by weight of the total weight of the starting oil is suflower seed oil, and from about 5% to about 20% by weight of the total weight of the starting oil is flaxseed oil. In another aspect, from about 50% to about 80% by weight of the total weight of the starting oil is olive oil, from about 10% to about 30% by weight of the total weight of the starting oil is flax seed oil, and from about 5% to about 20% by weight of the total weight of the starting oil is sunflower seed oil.

The process of making a product enriched in MAGs comprises a first step of hydrolyzing TAGs. By way of example but not limitation, the hydrolysis of TAGs may be carried out by a lipase such as lipase AY (Amano Enzymes, USA Elgin IL, USA), or any non-regiospecific lipase that cuts at the sn-1, sn-2 and sn-3 positions.

In some embodiments, the first step of hydrolyzing TAGs may be carried out at a temperature of about 30°C to 35°C. By way of example but not limitation, the first step of hydrolyzing TAGs may be carried out at a temperature of 30°C to 35°C, 31°C to 35°C, 32°C to 35°C, 33°C to 35°C, 34°C to 35°C, 30°C to 34°C, 31°C to 34°C, 32°C to 34°C, 33°C to 34°C, 30°C to 33°C, 31°C to 33°C, 32°C to 33°C, 30°C to 32°C, 31°C to 32°C, 30°C to 31°C, or 30°C, 31°C, 32°C, 33°C, 34°C, or 35°C.

In some embodiments, the first step of hydrolyzing TAGs may be carried out for about 14 hours to 24 hours. By way of example but not limitation, the first step of hydrolyzing TAGs may be carried out for 14 hours to 20 hours, 14 hours to 16 hours, 18 hours to 24 hours, 22 hours to 24 hours, 18 hours to 20 hours, or about 14 hours, 15 hours, 16 hours, 17 hours, 18 hours, 19 hours, 20 hours, 21 hours, 22 hours, 23 hours, or 24 hours.

In some embodiments, the first step of hydrolyzing TAGs results in hydrolysis of substantially all TAG. By way of example but not limitation, the first step of hydrolyzing TAGs results in hydrolysis of 94% to 100%, 95% to 100%, 96% to 100%, 97% to 100%, 98% to 100%, 99% to 100%, 94% to 99%, 95% to 99%, 96% to 99%, 97% to 99%, 98% to 99%, 94% to 98%, 95% to 98%, 96% to 98%, 97% to 98%, 94% to 97%, 95% to 97%, 96% to 97%, 94% to 96%, 95% to 96%, or 94% to 95% TAG, or at least 94%, 95%, 96%, 97%, 98%, 99%, or 100% of TAG.

The process of making a product enriched in MAGs comprises a second step of esterification with glycerol to enrich MAG oil content. By way of example but not limitation, this second step of esterification may be carried out by a lipase such as lipase G, (Amano Enzymes, USA Elgin IL, USA), or any regiospecific lipase that catalyzes esterification at the sn-1 position but does not effectively catalyze formation of the second or third ester on glycerol (to make DAGs and TAGs).

In some embodiments the second step of esterification with glycerol to enrich MAG oil content results in enrichment of MAGs in the product by about 70% to 95%. By way of example but not limitation, the MAG oil content may be enriched by 70% to 95%, 75% to 95%, 80% to 95%, 85% to 95%, 90% to 95%, 70% to 90%, 75% to 90%, 80% to 90%, 85% to 90%, 70% to 85%, 75% to 85%, 80% to 85%, 70% to 80%, 75% to 80%, 70% to 75%, or 70%, 75%, 80%, 85%, 90%, or 95%.

In some embodiments, the second step of esterification with glycerol may be carried out at a temperature of about 17°C to 23°C. By way of example but not limitation, the esterification with glycerol may be carried out at a temperature of 17°C to 23°C, 18°C to 23°C, 19°C to 23°C, 20°C to 23°C, 21°C to 23°C, 22°C to 23°C, 17°C to 22°C, 18°C to 22°C, 19°C to 22°C, 20°C to 22°C, 21°C to 22°C, 17°C to 21°C, 18°C to 21°C, 19°C to 21°C, 20°C to 21°C, 17°C to 20°C, 18°C to 20°C, 19°C to 20°C, 17°C to 19°C, 18°C to 19°C, 17°C to 18°C, or 17°C, 18°C, 19°C, 20°C, 21°C, 22°C, or 23°C.

In some embodiments, the second step of esterification with glycerol may be carried out for about 24 hours to 72 hours. By way of example but not limitation, the second step of esterification with glycerol may be carried out for 24 hours to 72 hours, 36 hours to 72 hours, 48 hours to 72 hours, 60 hours to 72 hours, 24 hours to 60 hours, 36 hours to 60 hours, 48 hours to 60 hours, 24 hours to 48 hours, 36 hours to 48 hours, 24 hours to 36 hours, or 24 hours, 30 hours, 36 hours, 42 hours, 48 hours, 54 hours, 60 hours, 66 hours, or 72 hours.

The process of making a product enriched in MAGs comprises a third step of lipase inactivation and phase separation.

The resulting product from the above embodiments results in a processed oil having a MAG content of equal to or greater than 40% by weight based on the total weight of the processed oil. In certain aspects, the MAG content is from about 40% to about 99% by weight based on the total weight of the processed oil. In certain aspects, the MAG content is from about 50% to about 99% by weight based on the total weight of the processed oil. In certain aspects, the MAG content is from about 60% to about 99% by weight based on the total weight of the processed oil. In certain aspects, the MAG content is from about 70% to about 99% by weight based on the total weight of the processed oil. In certain aspects, the MAG content is from about 80% to about 99% by weight based on the total weight of the processed oil. In certain aspects, the MAG content is from about 50% to about 80% by weight based on the total weight of the processed oil. In any of the above aspects, the TAG content is equal to or less than 5% by weight based on the total weight of the processed oil. In any of the above aspects, the TAG content is equal to or less than 4%, equal to or less than 3%, equal to or less than 2%, equal to or less than 1% by weight based on the total weight of the processed oil.

In some embodiments, the processed oil comprises a MAG content equal to or greater than 40% by weight of the total weight of the processed oil. By way of example but not limitation, the processed oil comprises a MAG content of about 40% to 95%, 50% to 95%, 60% to 95%, 70% to 95%, 80% to 95%, 90% to 95%, 40% to 90%, 50% to 90%, 60% to 90%, 70% to 90%, 80% to 90%, 40% to 80%, 50% to 80%, 60% to 80%, 70% to 80%, 40% to 70%, 50% to 70%, 60% to 70%, 40% to 60%, 50% to 60%, 40% to 50%, or about 40%, 45%, 50%, 55%, 60%, 65%, 70%, 75%, 80%, 85%, 90%, or 95% by weight of the total weight of the processed oil.

In some embodiments, the processed oil is either free of TAGs or comprises a TAG content that is equal to or less than 5% by weight of the total weight of the processed oil. By way of example but not limitation, the processed oil comprises a TAG content that is about 0% to 5%, 1% to 5%, 2% to 5%, 3% to 5%, 4% to 5%, 0% to 4%, 1% to 4%, 2% to 4%, 3% to 4%, 0% to 3%, 1% to 3%, 2% to 3%, 0% to 2%, 1% to 2%, 0% to 1%, or 0%, 1%, 2%, 3%, 4% or 5% by weight of the total weight of the processed oil. By way of further example but not limitation, the TAG content can be less than 5%, 4%, 3%, 2%, 1%, 0.5%, or 0.1%.

In some embodiments, the processed oil comprises non-oil ingredients derived from and naturally present in the oil source such that the non-oil ingredients are not added to the processed oil. By way of example but not limitation, such non-oil ingredients may include antioxidants such as tocopherols, which include alpha-tocopherol, beta-tocopherol, delta-tocopherol, gamma-tocopherol, alpha-tocotrienol, beta-tocotrienol, delta-tocotrienol, or gamma-tocotrienol, and other vitamins such as Vitamin K and structurally similar 2-methyl-1,4-naphthoquinone derivatives. In some embodiments, the antioxidant is selected from natural (*e.g*., mixed tocopherols or ascorbic acid) and synthetic (*e.g*., Butylated HydroxyAnisole or Butylated HydroxyToluene) antioxidants.

### EXAMPLES

### Example 1: Process of making a product enriched in MAG compared to starting TAG-rich oil.

The method of making a product enriched in MAG compared to the starting TAG-rich oil involves 3 key steps: (1) A mild enzymatically-catalyzed reaction to hydrolyze triglycerides (TAGs) in a sequence that converts the neutral oils to specific combinations of FFAs, MAGs, DAGs, and low residual TAGs; (2) an esterification with glycerol to generate predominantly high amounts of MAGs leaving low concentrations of FFAs; and (3) isolation of the modified lipid product; this is achieved by phase separation with or without the aid of a centrifuge.

### Step 1. Enzymatic Conversion of Triacylglycerols

**Preparation of the buffer solution.** A sodium citrate solution (100 mM, pH 5.8) was prepared in a stirred tank reactor. 11.1 L of deionized (DI) water was placed in the mixing vessel, the agitator was set to 200 RPM and 0.213 kg of citric acid (anhydrous) were added. After the powder was dissolved the pH was adjusted with sodium hydroxide solution to pH = 5.8 (about 0.121 kg). 220 mL was removed for subsequent enzyme preparation.

**Preparation of the enzyme solution.** In a separate 250 mL bottle, the enzyme solution was prepared under gentle stirring: 200 mL of the citrate buffer solution was placed in the mixing vessel. 10 g of AMANO Lipase AY was added and the bottle was shaken until the enzyme is dissolved.

**Conversion of Triglyceride Oil Mixture.** Three vegetable oils, Olive Oil, Flaxseed Oil and Sunflower seed Oil were added to the vessel to give a total of 10 kg of plant oil mixture. Vacuum was applied to drop the pressure to about 20 mmHg and degas the material (from any dissolved oxygen in particular). Agitation was set to 200 RPM and the mixture was heated to 33 °C and agitated for about 15 Min to remove any dissolved gas. The vacuum was then replaced by a Nitrogen gas atmosphere. Once the mixture was sufficiently dispersed, 220 mL of the enzyme preparation in buffer solution was added. Agitation was continued, and the reaction monitored for 24 hours until conversion to FFA was complete based on TLC analysis.

Reactor temperature was increased to 70° C and agitation resumed for 1 hour to inactivate the enzyme.

Agitation was stopped, and the phases separated in about 60 Minutes.

The aqueous (lower) phase was removed along with a small amount of the oil phase to ensure residual protein at the interface was eliminated.

**Step 2. Esterification with Glycerol.** Typically when FFA are re-esterified with glycerol they produce a mixture of MAGs, DAGs and TAGs. We found that by significantly reducing the temperature (below 25°C) and removing the water (by evaporation) as it is formed in the reaction, that the proportion of MAGs in the product can be highly enriched (at least 60% but as high as 95%). This was un-expected.

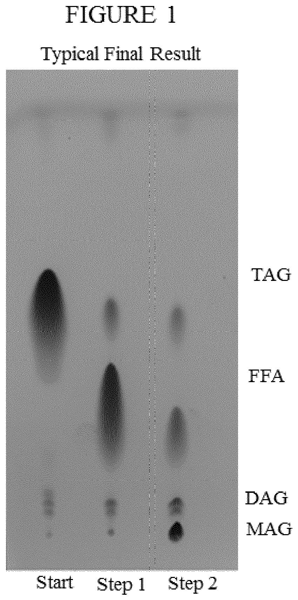

In the reaction, the reaction product from step 1 (about 10L) was cooled to about 30° C and agitated at 300 RPM. 10 kg of Food-grade glycerol were added to the Lipid mixture, and the temperature maintained at ~ 30° C. The mixture was agitated to generate a dispersion of the oil and glycerol. To dry the reaction mixture a vacuum was applied: First a vacuum (25 mmHg, Torr) was applied with receiver in place to collect water. Once evaporation of the residual water had stopped, 20g of Amano Lipase G dissolved in water (50 mL) was added to the reactor. The temperature was lowered to 23°C and the vacuum was changed to 5 mmHg using an oil diaphragm pump and a cold trap to collect the water. The mixture was stirred at 300 RPM at 23°C under vacuum for 72 hrs, at which time the vacuum was broken and the mixture was blanketed with nitrogen gas. The mixture was analyzed after 72 hrs with TLC to evaluate the conversion to MAGs as shown in FIGURE 1.

**Step 3. Lipase Inactivation and Phase Separation.** After the reaction was complete, the lipase was inactivated by heating the mixture (under a Nitrogen gas blanket) to 70°C for 1 hr. At this point the MAG oil and glycerol are well mixed and very hard to separate through traditional gravity or centrifugal methods. After considerable experimentation, we found that the lipid can be separated from the excess glycerol by adding 0.3%wt of salt (NaCl) to the reaction mixture under agitation. The product mixture was then allowed to cool to about 60°C and left without agitation for about 1 hrs.

The lipid oil phase separated from the remaining heavier glycerol phase. The glycerol phase was removed: it contains some salt, residual water and the dissolved inactivated enzyme which is contained in the visible interface. The glycerol phase can be reused after membrane filtration and should be kept for recycle. Tocopherol (Vitamin E) was added to give a concentration of 200 ppm (0.02% wt) of the product oil. The hydrolyzed oil (about 10 kg was ready for use and could be stored under nitrogen blanket).

**Product Storage.** The final hydrolyzed products was transferred to food-grade containers with an overlay of nitrogen gas for storage and transportation.

### Example 2: Characterization of oil produced in Example 1.

Reaction products and the overall process can be evaluated using Thin Layer Chromatography and Gas Chromatography.

**Thin-Layer Chromatography Testing.** Components of the oil samples were separated using TLC plates (Analtech Uniplate Silica Gel GHL with inorganic binder, 20 x 20 cm, 250 µm). The solvent was Hexane: Diethyl Ether: Acetic Acid (70:30:1) solution. Typical sample sizes were 3 µL. After the solvent front ran to near the top of the plate (~1 cm), plates were removed from the TLC tank and the solvent evaporated in a fume hood. The components were visualized with iodine vapors (at room temperature) in a TLC tank and relative intensities estimated by colorimetric imaging (Amersham 600 Imager). After 15 minutes in the tank, plates were removed and photographed. The intensity of the spots diminished after 30 - 60 minutes.

Physical properties determination was performed to establish product consistency, color, water content, fats & oils (miscible).

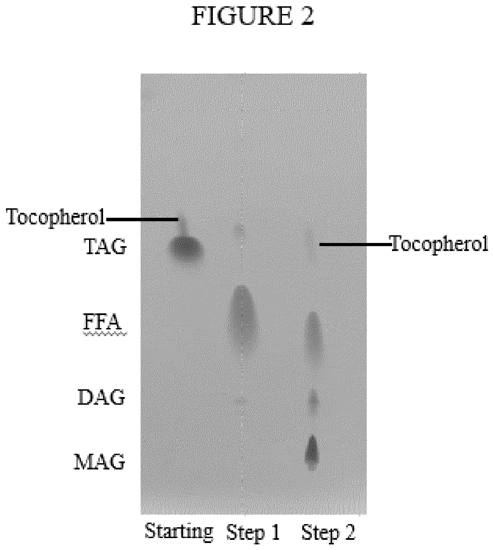

FIGURE 1 depicts the final results of steps 1 to 3 in the process of making a product enriched in MAG compared to starting TAG-rich oil as describe in Example 1. FIGURE 2 illustrates that tocopherol initially present in the olive oil is preserved following the steps described in Example 1. In FIGURE 2, the tocopherol spot can be seen tracking above the TAG spot in all three lanes.

**Fatty Acid Profile Testing - Gas Chromatography.** Lipid components including C10:0 Capric Acid, C12:0 Lauric Acid, C14:0 Myristic Acid, C16:0 Palmitic Acid, C18:0 Stearic Acid, C18:1 Oleic Acid, C18:2 Linoleic Acid, and C18:3 Alpha Linolenic Acid were analyzed after derivatization as the fatty acid methyl esters and compared to standards. For the derivatization, a sample (500 µl) was added to a 5-ml reaction tube containing 2 ml boron trifluoride solution (12% in methanol), 20 µl dimethoxypropane and 100 µl of a tridecanoic acid internal standard solution (10 mg/ml). The reaction tube was vortexed and incubated in a heating block at 60°C for 30 minutes.

The reaction tube was removed from the heating block and allowed to cool for 15 minutes. Then, 1 ml of distilled water was added to quench the reaction, followed by 1 ml of hexane. The reaction tube was vortexed for 60 seconds and the phases were allowed to separate for 3 minutes. The top (hydrophobic) phase was removed to a 1.5-ml tube containing about 50 mg sodium sulfate (anhydrous). After vortexing for 60 seconds, the 1.5-ml tube was centrifuged and ~500 µl of the clarified, dried hydrophobic phase was transferred to a gas chromatography sample vial.

Samples were analyzed using an Agilent 7890A gas chromatograph with Flame ionization detector and Agilent Openlab CDS Chemstation software. GC Column: Omegawax 100 (15m x 0.1 mm x 0.1 um) column. Results were converted to weight % by internal standard reference.

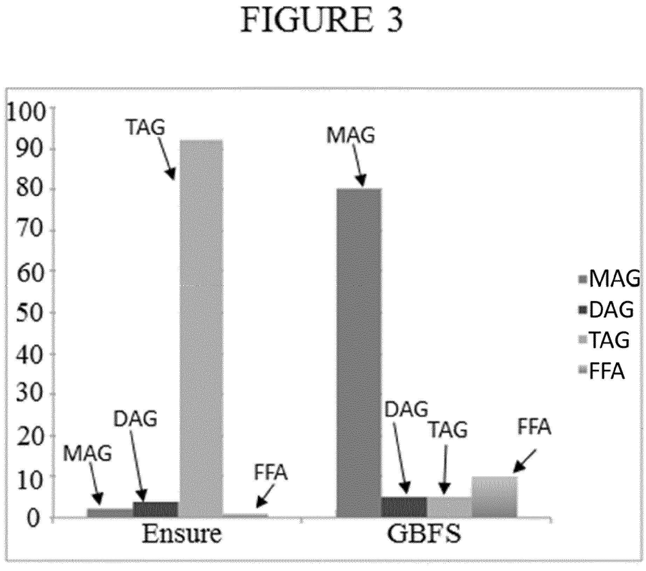

FIGURE 3 depicts the distribution of FFA, MAG, DAG, and TAG in Ensure and a GBFS product of the present disclosure. Percent FFA, MAG, DAG and TAG in the oils determined by Thin Layer Chromatography.

### Example 3: Another example of the process of making a product enriched in MAG compared to starting TAG-rich oil.

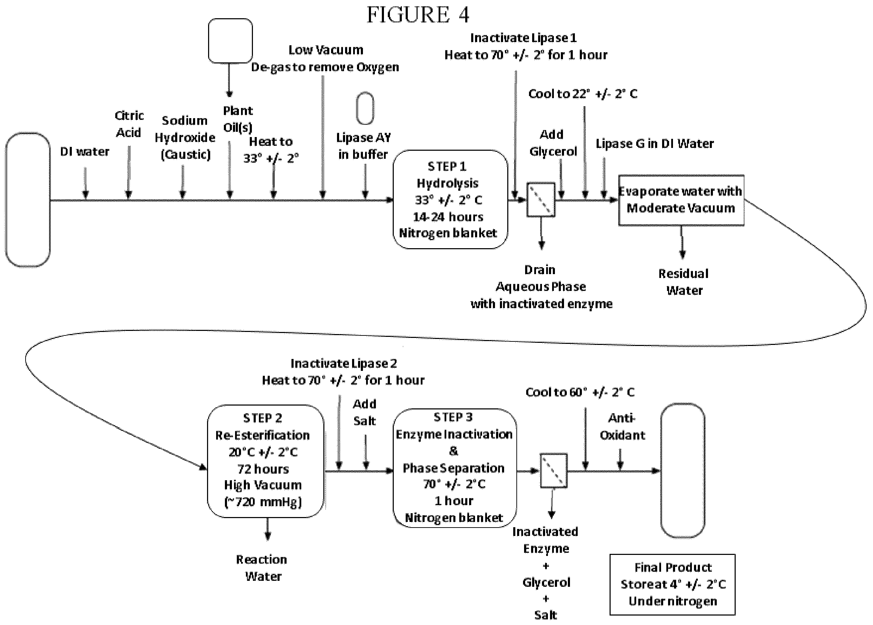

The following procedure is depicted by block flow diagram in FIGURE 4.

Plant oil was added to citric acid and sodium hydroxide (caustic) in DI water and heated to 33 °C +/- 2 °C. A low vacuum was applied to de-gas to remove oxygen. Lipase AY was added. Hydrolysis of the mixture was performed for 14 - 24 hours under a Nitrogen blanket at a temperature of 33 °C +/- 2 °C.

Lipase was inactivated at a temperature of 70 °C +/- 2 °C for 1 hour. The aqueous phase with inactivated enzyme was drained. Glycerol was added. Then the reaction was cooled to 22 °C +/- 2 °C. Then Lipase G was added and water was evaporated under a moderate vacuum.

Re-esterification was carried out for 72 hours under high vacuum (around 720 mmHg) at a temperature of 20 °C +/- 2 °C. Lipase G was inactivated at 70 °C +/- 2 °C for one hour and salt was added to the reaction.

Enzyme inactivation and phase separation was carried out under a Nitrogen blanket for 1 hour at 70 °C +/- 2 °C. The aqueous phase with inactivated enzyme, glycerol, and salt was drained. The reaction was cooled to 60 °C +/- 2 °C. Antioxidant was added. The final product was stored at 4 °C +/- 2 °C under nitrogen.

### Example 4: Ready-to-Drink formulation (not part of the invention) incorporating MAGs and hydrolyzed protein

A product of the present disclosure was produced as a conventional "milk shake" formulation that includes a source of fats, proteins, carbohydrates, vitamins and fiber in addition to the traditional surfactants and stabilizing agents typically found in these products. An individual serving was 250 ml. The ingredients as would appear on the ingredient label were as follows: Water, Organic Agave Syrup, Hydrolyzed Pea Protein, Hydrolyzed Oil Blend, Gum Arabic, Sunflower Lecithin, Xanthan Gum, Oligosaccharides, Potassium Sorbate, Sodium Benzoate, Instant Coffee, Natural Organic Vanilla Flavor, Vitamin C, Vitamin E Succinate, Vitamin A Palmitate, Niacinamide, D-Calcium Pantothinate, Pyrodoxine HCl, Thiamine HCl, Riboflavin, Vitamin D3, Folic Acid, Cyanocobalamin, Vitamin K2.

Carbohydrates were supplied as simple sugars (glucose and fructose) from fruit and agave syrups.

Protein in the product was partially hydrolyzed pea protein (PURIS Pea Protein 870H, World Food Processing LLC, Turtle Lake, WI 54889).

We also produced extensively hydrolyzed pea protein (EHP) with peptides in a size range more bio-available for transport across the intestinal wall. EHP was produced by further enzyme hydrolysis. For example, partially hydrolyzed pea protein (Puris Pea Protein 870H described above) protein was dissolved in 100 mM Phosphate buffer to a concentration of 25 mg/ml. Enzyme was added and the reaction incubated at 50° overnight. Three different commercial GRAS enzymes were evaluated: Alcalase, Thermoase and Flavourzyme.

The average size and distribution of peptides in the protein samples were evaluated using size exclusion chromatography. Samples were dissolved in 100 mM phosphate buffer, pH 6.8 to a concentration of 25 mg/ml and analyzed on a Shimadzu HPLC with UV detector (214 nm) using a Phenomenex Yarra 3 um SEC-2000 column (Eluted with 100 mM sodium Phosphate buffer (pH 6.8), Flow rate of 0.8 mL/min at room temperature). Samples were compared to a molecular weight standard (Phenomenex SEC standard part ALO-3042). Sizes were estimated using a calibration curve generated from known molecular weight standards. The average size of un-hydrolyzed pea protein was ~200 amino acids. The average size of the Puris Pea 870H partially hydrolyzed material was 34 amino acids, with a substantial amount in the 2-40 amino acid range, similar to other (whey-based) protein hydrolysate products.

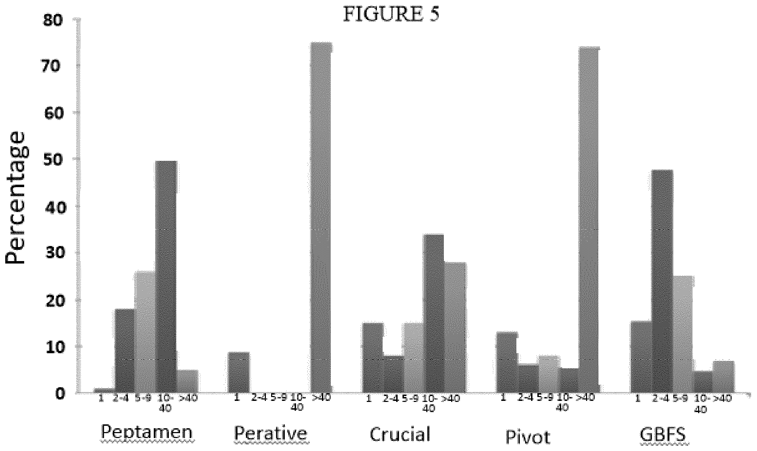

FIGURE 5 depicts the distribution of amino acids and peptides in ready-to-drink nutritional drinks as reported in E. Phillips *et al.,* 2005 Peptide-Based Formulas: the Nutraceuticals of Enteral Feeding? EPCN October:40-45, compared to the GBFS extensively hydrolyzed pea protein described above. FIGURE 5 depicts the percentage of amino acids (Y-Axis) of the size 1 amino acid, 2-4 amino acids, 9-10 amino acids, 10-40 amino acids, or greater than 40 amino acids in the products Oeotamen, Perative, Cricual, Pivot, and the GBFS of the present disclosure (X-Axis). The average size of the GBFS EHP is 3-4 amino acids, mostly 1 - 7 amino acids.

Fat is provided in the form of the re-structured Lipid MAG and were produced from a blend of olive oil (70%), sunflower oil (21%) and flax seed oil (9%) to provide the energy and other benefits of poly-unsaturated fatty acids (PUFA), omega-6 PUFA and omega-3 PUFA. The Omega-6/omega-3 ratio is ~4/1.

Gum Arabic, sunflower lecithin, and xanthan gum are common GRAS food ingredients used to provides structure and stability for the drink. Oligosaccharides provide non-digestable fiber. Potassium Sorbate and Sodium Benzoate are common food preservatives. Instant coffee and Vanilla provide flavor.

A vitamin package including fat soluble and water-soluble vitamins was included.

A prototype Product Nutritional label is shown in Table 1. In Table 1, ** Percentage Daily Values (% DV) are based on a 2,000 calorie diet. † Daily Value (DV) not established.

| **Supplement Facts Serving Size 250ml** | | | |
|---|---|---|---|
| | Amount Per Serving | | % DV ** |
| **Calories** | 379 | kcal | |
| | | | |
| **Total Fat** | 19 | g | 24% |
| **Cholesterol** | - | mg | 0% |
| | | | |
| **Total Carbohydrate** | 32 | g | 12% |
| Dietary Fiber | 2 | g | 7% |
| Sugars | 30 | g | |
| **Protein** | 20 | g | 40% |
| **Vitamin A** | 320 | mcg | 35% |
| **Vitamin C** | 480 | mg | 35% |
| **Vitamin D** | 7 | mcg | 35% |
| **Vitamin E** | 5 | mg | 35% |
| **Vitamin K** | 42 | mcg | 35% |
| **Thiamine** | 400 | mcg | 35% |
| **Riboflavin** | 450 | mcg | 35% |
| **Niacin** | 6 | mg | 35% |
| **Vitamin B6** | 600 | mcg | 35% |
| **Folate** | 132 | mcg | 35% |
| **Vitamin B12** | 1 | mcg | 35% |
| **Biotin** | 10 | mcg | 35% |
| **Pantothenic Acid** | 2 | mg | 35% |
| **Calcium** | 480 | mg | 35% |
| **Iron** | 6 | mg | 35% |
| **Phosphorus** | 450 | mg | 35% |
| **Chloride** | - | mg | † |
| **Sodium** | 175 | mg | 7% |
| **Potassium** | 1,600 | mg | 35% |

Sensory Evaluation. FFAs produced from the vegetable oil blend were found to be unpalatable by a taste panel. Surprisingly, the MAG oil produced from this blend was palatable and similar in the taste and texture of the original triglyceride oil. When the MAG oil was formulated into the RTD product described above, the flavor was acceptable and indistinguishable from similar commercial products with intact (un-digested) lipids and proteins.

### Example 5: Production of multiple batches of EMO

The timing of the process described in Example 1 for manufacturing EMO was evaluated to generate oil that was greater than 70% MAG, greater than >85% MAG + FFA and TAG content of 5% or less.

Table 2 depicts a scan of TLC plates illustrating the concentrations of MAG and FFA in the enzyme modified EMO produced from an olive oil/flax oil/sunflower oil blend (ratio of 7/2/1). In these experiments, step 2 was extended to 84 hours to establish upper limits on timing and temperature to avoid TAG formation.

| Experiment | TAG | DAG | MAG | FFA | FFA + MAG |
|---|---|---|---|---|---|
| 1 | 4 | 26 | 56 | 13 | 70 |
| 2 | 4 | 11 | 73 | 13 | 85 |
| 3 | 5 | 10 | 76 | 9 | 85 |

The reaction can be monitored in essentially real time with TLC analysis and stopped at any point during step 2 to yield to desired amounts of MAG, DAG, TAG and FFA. Reaction time of 72 hours for step 2 was found to be a practical and productive stopping point and illustrated in Example 6.

### Example 6: Evaluation of TAG-Free Enzyme Modified Almond Oil by NMR Analysis.

TAG-free EMO was produced using almond oil. Reaction conditions were as described in Example 1. Step 2 was 72 hours to minimize TAG production.

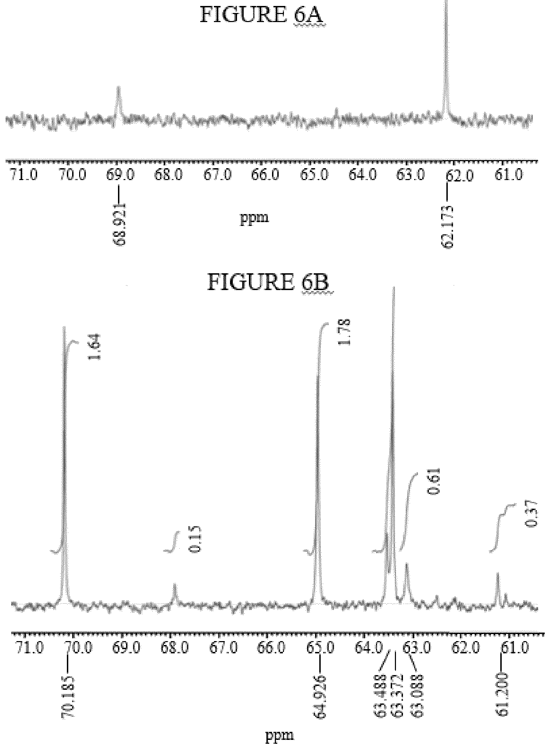

FIGURES 6A-6B depict the ¹³C-NMR analysis of the EMO produced from almond oil. FIGURE 6A illustrates that ¹³C-NMR signal associated with authentic TAG (tristerin) and the characteristic peaks at 62.173 ppm and 68.921 ppm. FIGURE 6B illustrates the ¹³C-NMR signal for the EMO. It shows that there are no discernable TAG signals. Integration of the actual signals indicates an acyl glycerol distribution between MAG:DAG of 88%:12%. Samples were analyzed on a JEOL model ECA600II NMR spectrometer operating at 600 MHz proton and 150 MHz carbon. Samples were made up in 5 mm tubes and run locked with CDCl3 at ambient temperature.

### Example 7: Clinical Testing of an EMO-based Ready-To-Drink-Shake (not part of the invention)

A study was designed to show that patients with EPI can consume food produced with the TAG-free oils we have produced, *without taking their enzyme supplement medication,* still absorb the lipids and produce TAGs in their serum, and not have the symptoms (bloating, cramps and steatorhea) associated with lipid consumption without taking PERT.

The clinical study was a single center, randomized, double-blind, cross-over trial assessing an EMO-based ready-to-drink shake (RTDS) for blood lipid levels, safety, tolerability and palatability compared to a standard nutritional supplement used concomitantly with pancreatic enzyme replacement therapy capsules ("PERT").

Patients came to the clinic after an overnight fast and standardized evening meal. Those in Arm 1 (10 patients) were administered the RTDS along with a PERT placebo and those in Arm 2 (10 patients) were administered standard nutritional supplement with PERT. Serial blood samples over 6 hours (0, 1, 2, 3, 4, 5, 6 hours) were obtained from patients in both study arms, with no repeat serving of the RTDS or standard nutritional supplement. Water could be consumed during the study.

Patients returned to the clinic for Treatment 2 (cross-over treatment) after overnight fast and standardized evening meal. Patients in Arm 1 were administered the standard nutritional supplement with PERT, while patients in Arm 2 were administered the RTDS along with PERT placebo. Serial blood samples over 6 hours (0, 1, 2, 3, 4, 5, 6 hours) were obtained from patients in both study arms, with no repeat serving of the RTDS or standard nutritional supplement.

The dosage of lipid was 0.5g/kg body weight. The lipid in the standard nutritional supplement was a blend of TAGs from canola, high oleic sunflower and corn oil. In the interventional drink the MAGs were produced from almond oil. The dosage of lipid for each patient was based upon well-established "Lipid Tolerance Tests", which are similar in design and scope to well-known glucose tolerance tests. The recommended dosage for these tests is 0.5 -1.0 g/kg administered over 20 -30 minutes. Samples were taken at the start of the test and hourly for 6-8 hours. Serum triglyceride levels were measured using standard laboratory methods.

The PERT dosage used in the study followed the manufacturer's recommended dosage guidelines of 2,500 iu of lipase activity per gram of fat ingested. This translated to 3-4 capsules during the crossover stage of the study.

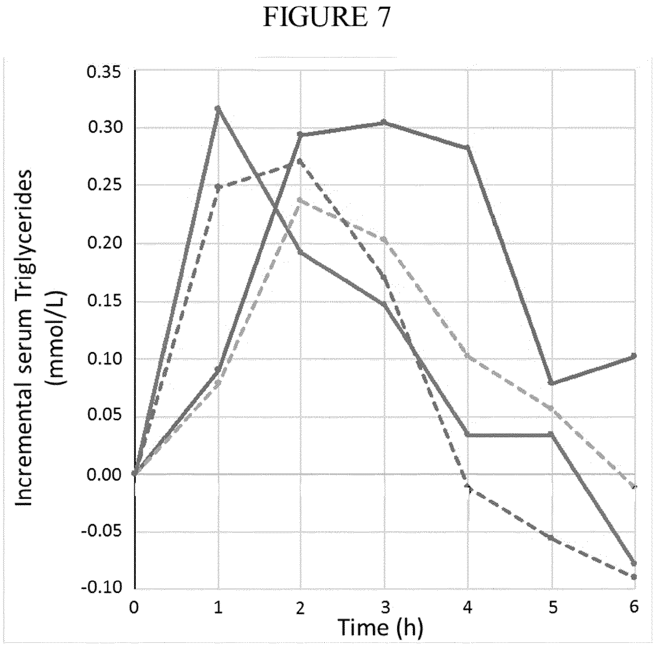

FIGURE 7 depicts the increase in serum triglycerides following ingestion of the EMO-based ready-to-drink shake in two patients. In FIGURE 7, the dashed lines represent patients that received 0.5 g per Kg of body weight of enzyme-modified almond oil and the solid lines represent patients that received 0.5 g per Kg of body weight of canola, high oleic sunflower and corn oil incorporated into the RTDS consumed over a half-hour period. FIGURE 7 illustrates that the enzyme-modified oil in the RTDS was absorbed by the patients and converted into serum triglycerides. The treatment was well tolerated.

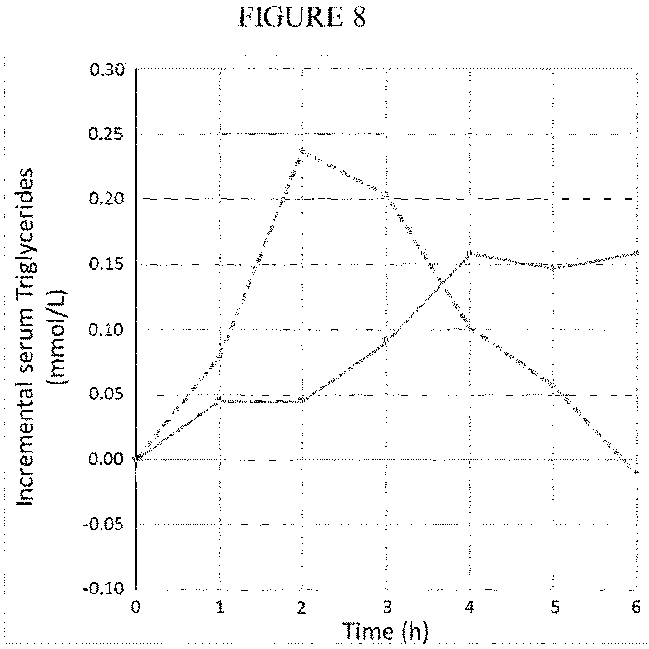

FIGURE 8 depicts the increase in serum triglycerides in another patient following ingestion of the test drinks. In FIGURE 8, the dashed line represents serum triglycerides following ingestion of MAG-based RTDS without PERT and the solid line represents standard of care RTDS with PERT. The patients received 0.5 g per Kg of body weight of enzyme-modified almond oil or 0.5 g per Kg of body weight of corn oil, incorporated in the RTDS consumed over a half-hour period. The treatment was well tolerated.

In this patient, the absorption of lipid and conversion to serum triglycerides was significantly faster following ingestion of the MAG-based RTDS without PERT than from the TAG-based (standard of care) product with PERT. This suggests that the patient was suffering from cholestasis (disruption of bile flow from the liver) in addition to EPI and not just suffering from lack of sufficient enzymes, and thus could not properly emulsify the canola, high oleic sunflower and corn oil in the standard of care drink. Emulsification of the oil into very small droplets is required to produce the surface area that the pancreatic lipase need hydrolyze the oil into the MAGs and FFA that are transported into the enterocytes. Bile acids from the liver are required for this activity. The TAGs in the standard of care drink could not be emulsified into the micro-emulsions required for optimal lipase activity in the small intestine, and thus serum triglycerides did not increase as quickly as with the MAG formulation, which does not require further emulsification or lipase activity.

### Example 8: High Calorie, PERT-free Ready-to-Drink Shake (not part of the invention)

A high calorie RTDS can be prepared as follows: Add DI water (~60% of final volume) into main mixing vessel, and heat water to ~60° C. Mix in Hydrolyzed Protein, then add Agave syrup. Use hand mixer to combine. In a separate container, combine warm (60° C) EMO and lecithin. When lecithin has dissolved, add EMO/Lecithin to aqueous phase & mix. Add additional water to achieve final weight (volume). Emulsify with high-shear blender. To prepare a very high calorie RTDS, add higher levels of the components.

Following production of the beverage base, various flavors, maskers and blockers can be added to produce unique products such as chocolate, vanilla, strawberry, etc.

This method can also be used to produce high calorie products in semi-solid formats such as puddings, mousses, "popsicles" and ice cream-like products using the beverage base recipe and adding viscosity altering agents such as xanthan gum and gum acacia.

Primary ingredients and nutritional values are shown in Table 3 for a high calorie (1.5 kcal/mL) RDTS.

| **Components with significant calories** | **Wt %** | **kcal/g** | **kcal/325 mL drink** | **kcal/mL** |
|---|---|---|---|---|
| EMO | 7.5 | 9 | 219 | 0.7 |
| Agave sugars | 14 | 4 | 182 | 0.6 |
| Hydrolyzed Protein pea fat | 5 | 4 | 65 | 0.2 |
| | 0.7 | 9 | 21 | 0.1 |
| Sunflower lecithin | 0.5 | 9 | 15 | 0.0 |
| **Total** | 28 | | 502 | 1.5 |

Primary ingredients and nutritional values are shown in Table 4 for a very high calorie (2.5 kcal/mL) RDTS.

| **Components with significant calories** | **Wt %** | **kcal/g** | **kcal/325 mL drink** | **kcal/mL** |
|---|---|---|---|---|
| EMO | 12 | 9 | 351 | 1.1 |
| Agave sugars | 20 | 4 | 260 | 0.8 |
| Hydrolyzed Protein pea fat | 10 | 4 | 130 | 0.4 |
| | 1.4 | 9 | 42 | 0.1 |
| Sunflower lecithin | 0.5 | 9 | 15 | 0.0 |
| **Total** | 44 | | 797 | 2.5 |

### Example 9: High Calorie, PERT-free Bar (not part of the invention)

A high calorie bar can be prepared as follows: Add DI water (~60% of final volume) into main mixing vessel, and heat water to ~60° C. Mix in Hydrolyzed Protein, then add Agave syrup. In a separate container, combine warm (60° C) EMO and lecithin with hand mixer. When lecithin has dissolved, add EMO/Lecithin to aqueous phase & mix. Add additional water to achieve final weight (volume).

Primary ingredients and nutritional values are shown in Table 5 for a high calorie, PERT-free bar.

| **Components with significant calories** | **Wt % in product** | **kcal/g** | **kcal/60 g bar** |
|---|---|---|---|
| EMO | 9 | 9 | 81 |
| Agave sugars | 15 | 4 | 60 |
| Hydrolyzed Protein pea fat | 19 | 4 | 76 |
| | 2.7 | 9 | 24 |
| Sunflower lecithin | 0.5 | 9 | 15 |
| **Total** | 46 | | 256 |

Following production of the bar base, various flavors, maskers and blockers can be added prior to baking to produce unique products such as chocolate, vanilla etc.

### Example 10: Manufacturing of a Ready-to-Drink-Shake (not part of the invention)

Add DI water into main mixing vessel, and heat water to ~60 +/- 2 °C. Once the water has reached 60° C, slowly add sugar syrup (such as agave or date syrup) with low agitation. Mix into solution. Weigh out the individual dry mass materials (Vitamin/Mineral mix and Hydrolyzed Protein) and combine in a separate container. Mix dry ingredients thoroughly. Slowly add mixed dry ingredients directly into main mixing vessel with sweep agitation at low agitation. Weigh out EMO in separate container. Heat the EMO to 60+/- 2 °C °C. Slowly add Sunflower lecithin to warm EMO and mix with moderate agitation (as needed) until the sunflower lecithin is completely mixed into solution. Slowly add EMO /sunflower lecithin mixture to main mixing vessel. Add distilled water to increase volume to 95% total fluid mass, return temperature at 70+/- 2 °C . Slowly add flavors and color to main mixing vessel. Slowly add stabilizer (such as acacia gum) to main mixing vessel. Mix solution for 20 minutes (to allow the viscosity to increase). Maintain temperature of solution at 70+/- 2 °C . QS solution with distilled water to final volume. Pass material through pressure drop homogenizer(s) to produce stable emulsions. Pasteurize or sterilize material. Cool material to room temperature. Fill product into packaging.

## Claims

1. A method for making a monoacylglycerol-enriched oil, comprising:
mixing a starting oil comprising triacylglycerols (TAGs), wherein the TAGs are in an amount greater than 50% by weight based on the total weight of the starting oil, a buffer solution and a first enzyme capable of hydrolyzing said TAGs to free fatty acids (FFAs) to yield a first reaction mixture;
allowing said first reaction mixture to react under conditions sufficient for said first enzyme to hydrolyze said TAGs for a first period of time to yield an aqueous phase and a first lipid reaction product comprising FFAs;
inactivating said first enzyme in said first lipid reaction product;
collecting said first lipid reaction product by removing it from the aqueous phase;
mixing said first lipid reaction product with a food-grade glycerol and a second enzyme capable of esterifying FFAs to form a second reaction mixture;
allowing said second reaction mixture to react for a second period of time to yield a second lipid reaction product comprising a lipid oil phase and a glycerol phase;
inactivating said second enzyme in said second lipid reaction product;
adding salt to the reaction product and separating the lipid oil phase from said glycerol phase; and
collecting said lipid oil phase.

2. The method of claim 1, wherein said starting oil is an oil derived from plant, animal or fish origin.

3. The method of claim 1, wherein said starting oil is a plant oil selected from the group consisting of olive oil, sunflower oil, corn oil, almond oil, rapeseed oil, palm oil, soybean oil, flaxseed oil, and mixtures thereof.

4. The method of any of claims 1-3, wherein said first enzyme is a lipase.

5. The method of any of claims 1-4, wherein said buffer solution is a sodium citrate solution.

6. The method of any of claims 1-5, wherein said first period of time is
(a) a period of time sufficient to hydrolyze at least 94% of the TAGs in said starting oil; and/or
(b) between about 14 hours and about 24 hours.

7. The method of any of claims 1-6, wherein said step of allowing said reaction mixture to react under conditions sufficient for said first enzyme to hydrolyze said TAGs is performed at a temperature between about 30°C and about 35°C.

8. The method of any of claims 1-7, wherein said second enzyme is a lipase.

9. The method of claim 8, wherein the first and second enzymes are each lipases, but different lipases.

10. The method of any of claims 1-9, wherein said second period of time is
(a) a period of time sufficient to result in enrichment of monoacylglyerides (MAGs) in the lipid oil phase of about 60% to 95%; and/or
(b) between about 24 hours and about 72 hours.

11. The method of any of claims 1-10, wherein said step of allowing said second reaction mixture to react for a second period of time to yield a lipid oil phase and a glycerol phase is performed at a temperature between about 17°C and 23°C.

12. The method of any of claims 1-11, wherein water is removed from the second reaction mixture during the second period of time.

13. The method of any of claims 1-12, wherein said step of inactivating said second enzyme is performed
(a) by heating said second lipid reaction product; or
(b) by heating said second lipid reaction product wherein said heating is performed at a temperature of at least 70°C for at least 1 hour.

14. The method of any of claims 1-13, wherein the lipid oil phase comprises MAGs, in an amount from about 40% to about 99% by weight based on the total weight of the lipid oil phase, and wherein the lipid oil phase either is free of TAGs or comprises TAGs in an about from about 0.1% to about 10% by weight based on the total weight of the lipid oil phase.

## Patentansprüche

1. Verfahren zur Herstellung eines mit Monoacylglycerin angereicherten Öls, umfassend:
Mischen eines Ausgangsöls, das Triacylglycerole (TAGs) umfasst, wobei die TAGs in einer Menge von mehr als 50 Gew.-%, auf der Grundlage des Gesamtgewichts des Ausgangsöls, enthalten sind, einer Pufferlösung und eines ersten Enzyms, das in der Lage ist, die TAGs zu freien Fettsäuren (FFAs) zu hydrolysieren, um ein erstes Reaktionsgemisch zu erhalten;
Ermöglichen, dass das erste Reaktionsgemisch unter Bedingungen reagiert, die ausreichend sind, damit das erste Enzym die TAGs über einen ersten Zeitraum hydrolysiert, um eine wässrige Phase und ein erstes Lipidreaktionsprodukt, das FFAs umfasst, zu erhalten;
Inaktivieren des ersten Enzyms in dem ersten Lipidreaktionsprodukt;
Auffangen des ersten Lipidreaktionsprodukts durch Entfernen aus der wässrigen Phase;
Mischen des ersten Lipidreaktionsprodukts mit einem lebensmitteltauglichen Glycerin und einem zweiten Enzym, das FFAs verestern kann, um ein zweites Reaktionsgemisch zu bilden;
Ermöglichen, dass das zweite Reaktionsgemisch über einen zweiten Zeitraum reagiert, um ein zweites Lipidreaktionsprodukt zu erhalten, das eine Lipidölphase und eine Glycerinphase umfasst;
Inaktivieren des zweiten Enzyms in dem zweiten Lipidreaktionsprodukt;
Hinzufügen von Salz zum Reaktionsprodukt und Trennen der Lipidölphase von der Glycerinphase; und
Auffangen der Lipidölphase.

2. Verfahren nach Anspruch 1, wobei es sich bei dem Ausgangsöl um ein Öl handelt, das aus Pflanzen, Tieren oder Fischen stammt.

3. Verfahren nach Anspruch 1, wobei das Ausgangsöl ein Pflanzenöl ist, das aus der Gruppe ausgewählt ist, die aus Olivenöl, Sonnenblumenöl, Maisöl, Mandelöl, Rapsöl, Palmöl, Sojaöl, Leinöl und Gemischen davon besteht.

4. Verfahren nach einem der Ansprüche 1 bis 3, wobei das erste Enzym eine Lipase ist.

5. Verfahren nach einem der Ansprüche 1 bis 4, wobei die Pufferlösung eine Natriumcitratlösung ist.

6. Verfahren nach einem der Ansprüche 1 bis 5, wobei der erste Zeitraum
(a) ein Zeitraum ist, der ausreicht, um mindestens 94 % der TAGs in dem Ausgangsöl zu hydrolysieren; und/oder
(b) zwischen etwa 14 Stunden und etwa 24 Stunden beträgt.

7. Verfahren nach einem der Ansprüche 1 bis 6, wobei der Schritt des Ermöglichen, dass das Reaktionsgemisch unter Bedingungen reagiert, die ausreichend sind, damit das erste Enzym die TAGs hydrolysieren kann, bei einer Temperatur zwischen etwa 30 °C und etwa 35 °C durchgeführt wird.

8. Verfahren nach einem der Ansprüche 1 bis 7, wobei das zweite Enzym eine Lipase ist.

9. Verfahren nach Anspruch 8, wobei das erste und das zweite Enzym jeweils Lipasen, aber unterschiedliche Lipasen sind.

10. Verfahren nach einem der Ansprüche 1 bis 9, wobei der zweite Zeitraum
(a) ein Zeitraum ist, der ausreicht, um eine Anreicherung von Monoacylglyceriden (MAGs) in der Lipidölphase von etwa 60 % bis 95 % zu bewirken; und/oder
(b) zwischen etwa 24 Stunden und etwa 72 Stunden beträgt.

11. Verfahren nach einem der Ansprüche 1 bis 10, wobei der Schritt des Ermöglichen, dass das zweite Reaktionsgemisch über einen zweiten Zeitraum reagiert, um eine Lipidölphase und eine Glycerinphase zu erhalten, bei einer Temperatur zwischen etwa 17 °C und 23 °C durchgeführt wird.

12. Verfahren nach einem der Ansprüche 1 bis 11, wobei während des zweiten Zeitraums Wasser aus dem zweiten Reaktionsgemisch entfernt wird.

13. Verfahren nach einem der Ansprüche 1 bis 12, wobei der Schritt des Inaktivierens des zweiten Enzyms durchgeführt wird
(a) durch Erhitzen des zweiten Lipidreaktionsprodukts; oder
(b) durch Erhitzen des zweiten Lipidreaktionsprodukts, wobei das Erhitzen bei einer Temperatur von mindestens 70 °C für mindestens 1 Stunde durchgeführt wird.

14. Verfahren nach einem der Ansprüche 1 bis 13, wobei die Lipidölphase MAGs in einer Menge von etwa 40 Gew.-% bis etwa 99 Gew.-%, auf der Grundlage des Gesamtgewichts der Lipidölphase, umfasst, und wobei die Lipidölphase entweder frei von TAGs ist oder TAGs in einer Menge von etwa 0,1 Gew.-% bis etwa 10 Gew.-%, auf der Grundlage des Gesamtgewichts der Lipidölphase, umfasst.

## Revendications

1. Procédé de fabrication d'une huile enrichie en monoacylglycérol, comprenant :
le mélange d'une huile de départ comprenant des triacylglycérols (TAG), dans lequel les TAG sont en une quantité supérieure à 50 % en poids sur la base du poids total de l'huile de départ, d'une solution tampon et d'une première enzyme apte à hydrolyser lesdits TAG en acides gras libres (AGL) pour produire un premier mélange réactionnel ;
le fait de permettre audit premier mélange réactionnel de réagir dans des conditions suffisantes pour que ladite première enzyme hydrolyse lesdits TAG pendant une première période de temps pour produire une phase aqueuse et un premier produit de réaction lipidique comprenant des AGL ;
l'inactivation de ladite première enzyme dans ledit premier produit de réaction lipidique ;
la collecte dudit premier produit de réaction lipidique en l'éliminant de la phase aqueuse ;
le mélange dudit premier produit de réaction lipidique avec un glycérol de qualité alimentaire et une deuxième enzyme apte à estérifier les AGL pour former un deuxième mélange réactionnel ;
le fait de permettre audit deuxième mélange réactionnel de réagir pendant une deuxième période de temps pour produire un deuxième produit de réaction lipidique comprenant une phase huileuse lipidique et une phase glycérol ;
l'inactivation de ladite deuxième enzyme dans ledit deuxième produit de réaction lipidique ;
l'ajout de sel au produit de réaction et la séparation de la phase huileuse lipidique de ladite phase glycérol ; et
la collecte de ladite phase huileuse lipidique.

2. Procédé selon la revendication 1, dans lequel ladite huile de départ est une huile dérivée de plante, d'origine animale ou de poisson.

3. Procédé selon la revendication 1, dans lequel ladite huile de départ est une huile végétale sélectionnée dans le groupe consistant en huile d'olive, huile de tournesol, huile de maïs, huile d'amande, huile de colza, huile de palme, huile de soja, huile de lin, et des mélanges de celles-ci.

4. Procédé selon l'une quelconque des revendications 1 à 3, dans lequel ladite première enzyme est une lipase.

5. Procédé selon l'une quelconque des revendications 1 à 4, dans lequel ladite solution tampon est une solution de citrate de sodium.

6. Procédé selon l'une quelconque des revendications 1 à 5, dans lequel ladite première période de temps est
(a) une période de temps suffisante pour hydrolyser au moins 94 % des TAG dans ladite huile de départ ;
et/ou
(b) entre environ 14 heures et environ 24 heures.

7. Procédé selon l'une quelconque des revendications 1 à 6, dans lequel ladite étape consistant permettre audit mélange réactionnel de réagir dans des conditions suffisantes pour que ladite première enzyme hydrolyse lesdits TAG est réalisée à une température comprise entre environ 30 °C et environ 35 °C.

8. Procédé selon l'une quelconque des revendications 1 à 7, dans lequel ladite deuxième enzyme est une lipase.

9. Procédé selon la revendication 8, dans lequel les première et deuxième enzymes sont chacune des lipases, mais des lipases différentes.

10. Procédé selon l'une quelconque des revendications 1 à 9, dans lequel ladite deuxième période de temps est
(a) une période de temps suffisante pour entraîner un enrichissement des monoacylglycérides (MAG) dans la phase huileuse lipidique d'environ 60 % à 95 % ; et/ou
(b) entre environ 24 heures et environ 72 heures.

11. Procédé selon l'une quelconque des revendications 1 à 10, dans lequel ladite étape de fait de permettre audit deuxième mélange réactionnel de réagir pendant une deuxième période de temps pour produire une phase huileuse lipidique et une phase glycérol est réalisée à une température comprise entre environ 17 °C et 23 °C.

12. Procédé selon l'une quelconque des revendications 1 à 11, dans lequel de l'eau est retirée du deuxième mélange réactionnel pendant la deuxième période de temps.

13. Procédé selon l'une quelconque des revendications 1 à 12, dans lequel ladite étape d'inactivation de ladite deuxième enzyme est réalisée
(a) en chauffant ledit deuxième produit de réaction lipidique ; ou
(b) en chauffant ledit deuxième produit de réaction lipidique dans lequel ledit chauffage est réalisé à une température d'au moins 70 °C pendant au moins 1 heure.

14. Procédé selon l'une quelconque des revendications 1 à 13, dans lequel la phase huileuse lipidique comprend des MAG, en une quantité d'environ 40 % à environ 99 % en poids par rapport au poids total de la phase huileuse lipidique, et dans lequel la phase huileuse lipidique est soit exempte de TAG, soit comprend des TAG d'environ 0,1 % à environ 10 % en poids par rapport au poids total de la phase huileuse lipidique.
